# EUROPÄISCHE PATENTANMELDUNG

(11) **EP 4 332 982 A1**
(43) Veröffentlichungstag der Anmeldung: **06.03.2024**
(21) Anmeldenummer: 22192637.1
(22) Anmeldetag: 29.08.2022
(51) Int. Cl.: G16H 30/40, G16H 40/60

(54) **STEUEREINRICHTUNG UND VERFAHREN ZUR STEUERUNG EINES MEDIZINTECHNISCHEN BILDGEBENDEN SYSTEMS**

(71) Anmelder: Siemens Healthineers AG, 91301 Forchheim (DE)
(72) Erfinder: Arroyo Camejo, Silvia Bettina, 90763 Fürth (DE); Wohlers, Julian, 91052 Erlangen (DE)
(74) Vertreter: Siemens Healthineers Patent Attorneys

(57) **Zusammenfassung**

Die Erfindung betrifft eine Steuereinrichtung (13) zur Steuerung eines medizintechnischen bildgebenden Systems (1), wobei die Steuereinrichtung (13) eine IQA-Einheit (20) umfasst, welche eine Anzahl von IQA-Algorithmen (21, 21a) enthält und zum Generieren eines Qualitätsmaßes (S, R) nach oder während der Applikation eines Steuerprotokolls (P) für eine Aufnahme dieser Bilddaten (B) durch das medizintechnische bildgebende System (1) ausgelegt ist, wobei das Qualitätsmaß (S, R) die Qualität der Bilddaten (B) bewertet, und wobei die Steuereinrichtung (13) dazu ausgelegt ist, basierend auf dem Qualitätsmaß (S, R) automatisch das medizintechnische bildgebende System (1) zu steuern.

Die Erfindung betrifft des Weiteren ein Verfahren zur Steuerung eines medizintechnischen bildgebenden Systems sowie ein entsprechendes medizintechnisches bildgebendes System.

## Beschreibung

Die Erfindung betrifft eine Steuereinrichtung und ein Verfahren zur Steuerung eines medizintechnischen bildgebenden Systems, z. B. eines Magetresonanztomographie-Systems (MRT-Systems), eines Computertomographie-Systems (CT-Systems) oder eines Positron-Elektron-Tomographie-Systems (PET-Systems). Die Erfindung basiert dabei insbesondere auf automatisierten Image Quality Assessment Algorithmen (IQA-Algorithmen), die ein Qualitäts-Feedback für den Benutzer eines Diagnosescanners liefern.

Die Magnetresonanztomographie (MRT), Computertomographie (CT) und Positron-Elektron-Tomographie (PET) sind leistungsstarke und vielseitige Untersuchungsmethoden, die es dem Arzt ermöglichen, strukturelle und funktionelle Informationen aus dem Inneren des Körpers eines Patienten zu erhalten. Die entsprechenden Systeme bieten einem Benutzer oftmals eine Vielzahl von Einstellmöglichkeiten für eine Bildgebung. Beispielsweise stehen bei einem MRT-System einem Benutzer eine Vielzahl von Bildgebungssequenzen und Protokollparametern zur Verfügung, um den MR-Bildkontrast (MR: Magnetresonanz) anzupassen, d.h. wie sich bestimmte Gewebeeigenschaften oder Perfusions- und Diffusionsverhalten von Flüssigkeiten im Bildeindruck manifestieren. Solche Einstellmöglichkeiten ermöglichen einem Mediziner nach sehr spezifischen Merkmalen zu suchen, um einen Verdacht auf eine Pathologie zu bestätigen oder abzulehnen.

Damit ein Radiologe diese diagnostische Aufgabe zuverlässig durchführen kann, ist eine Mindestbildqualität erforderlich. Die erforderliche minimale Bildqualität kann von der klinischen Indikation des Patienten, der vermuteten Pathologie, der Körperregion und/oder anderen Faktoren abhängen. Wenn diese minimale Bildqualität nicht erreicht wird, und dies nicht bemerkt wird, während sich der Patient noch im Scanner bzw. der Aufnahmeeinheit befindet, werden häufig Patientenrückrufe erforderlich, was dem Patienten Unannehmlichkeiten bereitet und Zeit für Scanner, Techniker und Radiologen verschwendet. Kann der Radiologe aufgrund unzureichender Bildqualität bei der ersten Scan-Untersuchung keine Diagnose stellen, birgt ein Patientenrückruf das Risiko einer verspäteten Diagnose. Darüber hinaus reduzieren Bildqualitätsprobleme den gesamten Lesedurchsatz, da der Lesevorgang unterbrochen wird, und erhöhen das Risiko von Fehldiagnosen oder fehlenden Befunden.

Normalerweise führt der Benutzer des Scanners bzw. der Aufnahmeeinheit (d.h. der Techniker) unmittelbar nach der Bilderfassung eine manuelle Bildqualitätsprüfung durch, die in besonderen Fällen durch einen Radiologen unterstützt wird. Eine zuverlässige Beurteilung der Bildqualität erfordert vom Techniker ein gewisses Maß an Fachwissen und praktischer Erfahrung sowie ein Verständnis dafür, wie die spezifische Indikation, die vermutete pathologische Körperregion usw. die erforderliche minimale Bildqualität verändert. Dazu durchlaufen erfahrene Techniker eine jahrelange Ausbildung und jahrelange praktische Erfahrung, bevor sie diese Aufgabe für alle Bildaussagen (z. B. MR-Kontraste), Indikationen und Körperregionen zuverlässig meistern. Unerfahrene Techniker und Techniker, die unter Zeitdruck arbeiten, sind möglicherweise nicht in der Lage, diese manuelle Bildqualitätssicherung mit hoher Genauigkeit durchzuführen. Folgen einer ungenauen Bildqualitätssicherung sind beispielsweise Underscanning (keine Erkennung einer unzureichenden Bildqualität bei der Untersuchung und Rückruf des Patienten zur Nachuntersuchung) oder Overscanning (erneutes Scannen von Aufnahmeschritten mit der Absicht, eine bessere Bildqualität zu erhalten, obwohl das erste erzeugte Bild eine ausreichende Bildqualität für diagnostisches Lesen besaß). Beide Fälle verursachen unnötige Scanzeit, verschwenden die Zeit des Radiologen, verursachen Unannehmlichkeiten für den Patienten. Ein Underscanning kann sogar das Risiko einer verspäteten Diagnose bewirken.

Eine weitere Herausforderung, die insbesondere für Gesundheitsdienstleister mit einer größeren Scannerflotte und/oder mehreren Einrichtungen (z. B. ein sogenanntes Narbe-Speichen-Modell) relevant ist, ist die Variabilität der Bildqualität. Die Variabilität der Bildqualität ergibt sich aus Unterschieden im Scannermodell, der Magnetfeldstärke, unterschiedlichen Protokolleinstellungen, Patienteneigenschaften (Implantate vorhanden, groß, adipös), Patientenverhalten (kooperiert bei Atemkommandos, bewegt sich viel) und nicht zuletzt dem Erfahrungsniveau und den persönlichen Vorlieben des Scannerbenutzers. Die Etablierung eines flottenweiten Bildqualitätsstandards für Scanner ist daher eines der wichtigsten Ziele moderner, großer Gesundheitsdienstleister. Dazu bedarf es eines standardisierten und idealerweise automatisierten Verfahrens zur Beurteilung der Qualität jedes Bildes bereits bei der Aufnahme, um eine gewisse Mindestbildqualität zu gewährleisten.

Es ist eine Aufgabe der vorliegenden Erfindung, eine Steuereinrichtung und ein Verfahren zur Steuerung eines medizintechnischen bildgebenden Systems, z. B. eines MRT-Systems, eines CT-Systems oder eines PET-Systems anzugeben, mit der die oben beschriebenen Nachteile vermieden werden und insbesondere eine automatisierte Qualitätsprüfung möglich ist.

Diese Aufgabe wird durch eine Steuereinrichtung nach Patentanspruch 1, ein Verfahren gemäß Patentanspruch 9 sowie ein medizintechnisches bildgebendes System nach Patentanspruch 12 gelöst.

Eine erfindungsgemäße Steuereinrichtung dient zur Steuerung eines medizintechnischen bildgebenden Systems, welches Bildaufnahmen mittels Steuerprotokollen durchführen sollte. Damit ist insbesondere ein Magetresonanztomographie-System (MRT-System), ein Computertomographie-System (CT-System) oder ein Positron-Elektron-Tomographie-System (PET-System) sowie Kombinationen von diesen gemeint, da die Erfindung für diese Systeme besonders vorteilhaft ist. Was eine Kombination betrifft, ist die Erfindung besonders für hybride Systeme vorteilhaft wie z. B. PET/CT oder PET/MR. Hier ist es häufig besonders schwierig qualifiziertes Personal mit viel Erfahrung zu finden, weil es sehr spezielle, seltene Hybrid-Modalitäten sind, die besonders viel Erfahrung in beiden Einzelmodalitäten erfordern. Jedoch kann die Erfindung auch für andere medizintechnischen bildgebenden Systeme vorteilhaft sein, sofern diese eine automatisierte Aufnahme vieler Daten mittels Steuerprotokollen vornehmen. Auch wenn die Erfindung für den Bereich MRT besonders vorteilhaft ist, da die Aufnahmen dort besonders lange dauern (und die nachfolgenden Ausführungen gerne im Lichte einer MRT-Untersuchung gelesen werden können), kann die Erfindung durchaus auch im Bereich CT oder PET eine vorteilhafte, praktische Anwendung finden, da auch dort ein Scan manchmal mehrere Male wiederholt werden muss, bis ein diagnostisches Bild entsteht und Nachuntersuchungen aufgrund mangelhafter Aufnahmen auch dort nachteilhaft sind.

Die Steuereinrichtung umfasst eine IQA-Einheit, welche eine Anzahl von IQA-Algorithmen enthält und zum Generieren eines Qualitätsmaßes nach oder während der Applikation eines Steuerprotokolls für eine Aufnahme dieser Bilddaten durch das medizintechnische bildgebende System ausgelegt ist, wobei das Qualitätsmaß die Qualität der Bilddaten bewertet, und wobei die Steuereinrichtung dazu ausgelegt ist, basierend auf dem Qualitätsmaß automatisch das medizintechnische bildgebende System zu steuern.

Die Abkürzung "IQA" steht für "Image Quality Assessment" und wird im Rahmen der Bildverarbeitung auch im Deutschen verwendet. Image Quality Assessment ist im Stand der Technik bereits weitgehend bekannt. Bilddaten werden in einen IQA-Algorithmus (oft mit IQAA abgekürzt) eingegeben, dort verarbeitet und als Ergebnis wird ein Maß für die Qualität der Bilddaten ausgegeben. Es wird also im Nachfolgenden Text der englische Begriff Image Quality Assessment bzw. dessen Abkürzung IQA verwendet.

Auch wenn nachfolgend die IQA-Algorithmen noch genauer beschrieben werden, ist dies doch weitestgehend Stand der Technik. Die Erfindung betrifft auch nicht die Ermittlung der Qualität an sich, sondern die automatisierte Steuerung eines bildgebenden Systems unter Verwendung der Ergebnisse von IQAAs. Das Maß für die Qualität der Bilddaten eines IQAA kann bereits das Qualitätsmaß sein, jedoch ist oder enthält das Qualitätsmaß bevorzugt ein weiterverarbeitetes Ergebnis eines IQAA oder mehrerer IQAA. Es sollte beachtet werden, dass nicht notwendigerweise nur Bilddaten in einen IQAA eingegeben werden müssen, sondern auch Sensordaten eingegeben werden können, aus denen eine Bildqualität abgeleitet werden kann. Beispielsweise kann ein IQAA auch Bewegungsdaten des aufgenommenen Patienten erhalten und beim Überschreiten eines erlaubten Bereichs für Bewegungen die Qualität als mangelhaft einstufen, weil sich der Patient zu viel bewegt hat.

Die IQA-Einheit dient zum Generieren des Qualitätsmaßes aus den Bilddaten oder aus Sensordaten, aus denen eine Bildqualität abgeleitet werden kann und stellt auf die Erfindung bezogen das Herz der Steuereinrichtung dar. Hierzu ist aber anzumerken, dass die Steuereinrichtung im Grunde auch für die Steuerung des bildgebenden Systems, zumindest dessen Scanner, verwendet werden könnte und dafür weitere, dazu wichtige, Komponenten enthalten kann.

Die IQA-Einheit umfasst eine Anzahl von IQA-Algorithmen und ggf. weitere Komponenten, wie z. B. eine Aggregations-Einheit zum Zusammenfassen von Ergebnissen der Anzahl von IQA-Algorithmen oder anderen Resultaten, eine Scoring-Einheit zum Generieren eines numerischen Werts für das Qualitätsmaß (z. B. von 0% bis 100%) und/oder eine Rating-Einheit zum Generieren eines qualitativen Werts für das Qualitätsmaß (z. B. "gut" oder "schlecht"). In einem sehr einfachen Beispiel kann ein IQA-Algorithmus, welcher ein numerisches Maß für die Qualität aus Bilddaten oder aus Daten eines Bewegungssensors oder aus Daten eines EKG generiert, bereits alleine die IQA-Einheit darstellen.

Es sei darauf hingewiesen, dass die Bilddaten sowohl Rohdaten als auch Daten rekonstruierter Bilder sein können (oder eine Kombination von beidem). Bilddaten können auch aus einer Aufnahme im Rahmen der Spektroskopie stammen. Im Grunde hängt dies lediglich von den verwendeten IQA-Algorithmen ab bzw. dem Datenformat, welches in die IQA-Algorithmen eingegeben werden muss. Werden z. B. mehrere IQA-Algorithmen verwendet, von denen ein Teil Rohdaten als Input benötigt und ein anderer Teil rekonstruierte Bilder, so können die Bilddaten auch sowohl Rohdaten als auch Daten rekonstruierter Bilder enthalten. Die Bilddaten können beispielsweise MR-Daten, CT-Daten oder PET-Daten sein.

Das Qualitätsmaß kann nach oder während der Applikation eines Steuerprotokolls für eine Aufnahme der Bilddaten generiert werden, wobei die jeweils aufgenommenen Bilddaten zur Generierung des Qualitätsmaßes verwendet werden. Es können aber wie gesagt auch Sensordaten verwendet werden. Beispielsweise können Bilddaten einer Schicht bereits zur Generierung eines Qualitätsmaßes dienen, während gerade eine nachfolgende Schicht aufgenommen wird. Sollte die untersuchte Schicht mangelhaft sein, dann kann die Aufnahme sofort gestoppt und wiederholt oder die mangelhafte Schicht erneut vermessen werden. Entsprechendes gilt für Sensordaten. Es ist bevorzugt, dass das Qualitätsmaß die Qualität der Bilddaten gemäß den bekannten Grundsätzen des Image Quality Assessment bewertet. Es kann aber auch anderen, vorher festgelegten Kriterien unterworfen sein, z. B. dass bei einer starken Bewegung des Patienten oder einer langen Bewegungsphase das Qualitätsmaß eine schlechte Qualität anzeigt. Im Rahmen des Qualitätsmaßes können insbesondere Qualitätsbewertungen und/oder diagnostische Bildqualitätsklassen ermittelt werden.

Wichtig ist, dass die Steuereinrichtung so gestaltet ist, dass sie automatisch das medizintechnische bildgebende System steuern kann, wozu sie selbstverständlich das Qualitätsmaß auch auswerten sollte. Eine solche Steuerung kann unterschiedlicher Natur sein, erfolgt jedoch stets automatisiert, also ohne Zutun eines Benutzers. Beispielsweise kann eine Steuerung ein Abbruch oder eine Wiederholung der Aufnahme oder von Teilen der Aufnahme umfassen. Ebenso kann die Steuerung eine automatisierte Ausgabe einer Warnung oder einer Information über das Qualitätsmaß umfassen. Sie kann auch eine Patienteninstruktion umfassen, z. B. die Anweisung "Bleiben Sie bitte still liegen!". Die Steuerung kann sogar die Anpassung von Aufnahmeparametern für nachfolgende Teile der Aufnahme oder eine neue Aufnahme umfassen.

Ein erfindungsgemäßes Verfahren zur Steuerung eines medizintechnischen bildgebenden Systems, insbesondere eines MRT-Systems, eines CT-Systems oder eines PET-Systems, mit einer erfindungsgemäßen Steuereinrichtung umfasst die folgenden Schritte:
- Applizieren eines Steuerprotokolls für eine Aufnahme von Bilddaten mit einer Aufnahmeeinheit des medizintechnischen bildgebenden Systems,
- automatisches Ermitteln eines Qualitätsmaßes für die Qualität der aufgenommenen Bilddaten mittels der IQA-Einheit der Steuereinrichtung,
- Steuern des medizintechnischen bildgebenden Systems basierend auf dem Qualitätsmaß.

Das Applizieren eines Steuerprotokolls für eine Aufnahme von Bilddaten mit einer Aufnahmeeinheit des medizintechnischen bildgebenden Systems ist im Stand der Technik bekannt und wird standardmäßig bei medizintechnischen Bildaufnahmen verwendet, z. B. bei MRT-Systemen, CT-Systemen oder PET-Systemen.

Das automatische Ermitteln des Qualitätsmaßes wurde vorangehend bereits beschrieben, wird aber auch im nachfolgenden Text thematisiert.

Auch das Steuern des medizintechnischen bildgebenden Systems basierend auf dem Qualitätsmaß wurde bereits beschrieben. Es ist dabei bevorzugt, dass in dem Fall, dass das ermittelte Qualitätsmaß außerhalb eines vorgegebenen Bereichs liegt, eine Warnung von dem medizintechnischen bildgebenden System an einen Benutzer ausgegeben wird und/oder die Aufnahme der Bilddaten mit geänderten Aufnahmeparametern wiederholt wird und bevorzugt basierend auf dem Qualitätsmaß eine Anzahl einzelner Schichten oder Segmente neu erfasst und in ursprünglich aufgenommene Bilddaten eingebettet werden. Was die geänderten Aufnahmeparameter betrifft, ist davon insbesondere auch umfasst, dass eine andere Aufnahmestrategie verwendet werden kann, also andere Steuerprotokolle ausgewählt werden können. Mit Änderungen von Aufnahmeparametern kann insbesondere umfasst sein, dass eine Wiederholung des letzten Steuerprotokolls eingeleitet wird (ggf. mit ein oder mehreren geänderten internen Protokoll-Parametern), eine Ausführung eines alternativen (besser geeigneten) Steuerprotokolls, ein Wechsel in eine andere (besser geeignete) Scan-Strategie, z. B. mehrere alternative Steuerprotokolle, bei getriggerten Protokollen, eine neue Kalibration des Triggers oder ein Wechsel der Trigger-Quelle. Es kann dabei eine Wiederholung der gesamten Aufnahme oder auch nur die Wiederholung der Aufnahme einzelner Bildausschnitte bzw. Schichten eingeleitet werden.

Somit ermöglicht die Erfindung eine Möglichkeit zur nahtlosen Integration von Bildqualitäts-Bewertungsalgorithmen (IQA-Algorithmen) in den Bilderfassungsprozess, mit dem Ziel, die diagnostische Qualität eines Bildes während oder kurz nach der Erfassung zu bewerten und sofort entsprechende Aktionen zur Warnung oder zur Verbesserung in die Wege zu leiten, falls eine unzureichende Bildqualität festgestellt wurde. Das System ermöglicht mit einer Auswahl verschiedener IQA-Algorithmen eine modulare Integration von IQAAs in einen diagnostischen Bildgebungsscanner.

Ein erfindungsgemäßes medizintechnisches bildgebendes System ist insbesondere ein MRT-System, ein CT-System oder ein PET-System und umfasst eine erfindungsgemäße Steuereinrichtung und ist bevorzugt zur Durchführung eines erfindungsgemäßen Verfahrens ausgelegt. Bevorzugt umfasst das medizintechnische bildgebende System eine Anzeigeeinheit, wobei die Steuereinrichtung dazu ausgelegt ist, das Qualitätsmaß, z. B. einen numerischen Wert der Qualität, oder auf dem Qualitätsmaß basierende Bewertungen, z. B. "Gut" / "Schlecht", darzustellen und oder andere Ergebnisse der IQA-Einheit darzustellen, insbesondere (im Rahmen der IQA) rekonstruierte Bilder.

Die Erfindung kann insbesondere in Form einer Rechnereinheit, insbesondere in einer Steuereinrichtung für ein medizintechnisches bildgebendes System, mit geeigneter Software realisiert sein. Die Rechnereinheit kann z. B. hierzu einen oder mehrere zusammenarbeitende Mikroprozessoren oder dergleichen aufweisen. Insbesondere kann sie in Form von geeigneten Softwareprogrammteilen in der Rechnereinheit realisiert sein. Eine weitgehend softwaremäßige Realisierung hat den Vorteil, dass auch schon bisher verwendete Rechnereinheiten auf einfache Weise durch ein Software- bzw. Firmware-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Rechnereinheit ladbar ist, mit Programmabschnitten, um alle Schritte des erfindungsgemäßen Verfahrens auszuführen, wenn das Programm in der Rechnereinheit ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z. B. Hardware-Schlüssel (Dongles etc.) zur Nutzung der Software, umfassen. Zum Transport zur Rechnereinheit und/oder zur Speicherung an oder in der Rechnereinheit kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

IQA-Algorithmen können basierend auf einfacher Bildverarbeitung, Signalverarbeitung, klassischen Computer-Vision Methoden oder Machine-Learning-/Deep-Learning-Methoden entwickelt werden.

Als IQAAs geeignete Bildverarbeitungsalgorithmen wären z. B. Algorithmen zur Signal-Rausch-Berechnung und/oder zur Kontrast-Rausch Berechnung.

Als IQA-Algorithmen geeignete klassische Computer-Vision Methoden wären z. B.
- Erkennungs- oder Detektionsalgorithmen zum Auffinden von Merkmalen, die mit einer bestimmten Art von Bildqualitätsproblemen verbunden sind (z. B. die Welligkeitsstruktur in durch Bewegungsartefakte beschädigten Bildern oder sich wiederholende Strukturen eines Aliasing-Artefakts), oder
- Signalverarbeitung von Zeitreihen, die von Sensoren oder (Teil-)Bilddaten-Messungen erstellt wurden, die direkt oder indirekt verschiedene Bewegungsmerkmale eines Patienten messen, z. B. Herzschlag, Atemzyklus, Darmbewegung oder Körperbewegung, und bestimmen, mit welcher Wahrscheinlichkeit die analysierten Zeitreihen mit Bildqualitätsproblemen korrelieren.

Auf maschinellem Lernen oder "Tiefem Lernen" (Deep Learning) basierende IQA-Algorithmen können so trainiert werden, dass eine allgemeine Bildqualität oder die Identifizierung spezifischer Artefakte (z. B. Bewegungsartefakte oder Gibbs-Ringing) in Form
- eines Klassifikationsalgorithmus, der N Bildqualitätsbewertungsklassen bereitstellt. Beispiele dafür wären ein binärer Klassifikator mit den beiden Labels "gut" und "schlecht" für eine diagnostische Bildqualität oder ein mehrklassiger Klassifikator mit z. B. den fünf Labels "sehr gut", "gut", "befriedigend", "ausreichend", "ungenügend" oder
- von Regressionsalgorithmen, die eine kontinuierliche Bewertung der Bildqualität innerhalb eines endlichen Wertebereichs liefern, z. B. im Intervall [0,1], wobei 0 die schlechteste und 1 die bestmögliche Bewertung ist
erfolgt.

IQA-Algorithmen können so gestaltet werden, dass sie mit verschiedenen Arten von Bilddaten arbeiten, einschließlich Rohdaten, verschiedenen Bildkontrasten und Messkonfigurationen. All diese Daten können in den Bilddaten enthalten sein. Ein wichtiger Anwendungsfall ist das Image Quality Assessment von unbearbeiteten 2D oder 3D K-Raum-Bildern bei MR-Aufnahmen, räumlichen 2D- oder 3D-Bildern oder für eine zeitaufgelöste Bildgebung basierend auf räumlichen 2D-Bildern mit zeitlichen 1D-Bildern oder räumlichen 3D-Bildern mit zeitlichen 1D-Bildern. Für die Standardverwendung ist es eine bevorzugte Konfiguration, dass ein IQA-Algorithmus ein Qualitätsmaß pro generierter DICOM-Serie generiert, da dies die typische Granularität ist, in der Bilder vom Radiologen gelesen oder verworfen werden. Die Abkürzung "DICOM" steht dabei für "Digital Imaging and Communications in Medicine" (deutsch: Digitale Bildverarbeitung und Kommunikation in der Medizin) und stellt einen wichtigen Standard dar.

Ohne Einschränkung der Allgemeinheit könnten sich spezielle Anwendungsfälle von IQA-Algorithmen auf die Analyse der Qualität von MR-Spektroskopiemessungen, quantitativen T1- oder T2-Karten oder MR-Fingerprinting-Karten erstrecken.

Eine weitere Variante sind IQAAs, die eine Qualitätsbewertung auf Unterserienebene (einzelne Schicht oder sogar ein Segment eines DICOM) bieten. Basierend auf dieser Bewertung könnten die beschädigte(n) Schicht(en) oder Segment(e) z. B. einzeln neu erfasst und in die ursprüngliche Schicht oder Serie eingebettet werden.

Gemäß einer bevorzugten Steuereinrichtung ist daher zumindest ein IQA-Algorithmus aus einer Gruppe ausgewählt, welche die folgenden Elemente umfasst:
- IQA-Algorithmen zur Signal-Rausch-Berechnung oder Kontrast-Rausch Berechnung,
- Detektionsalgorithmen zum Auffinden von Merkmalen, die mit einer bestimmten Art von Bildqualitätsproblemen verbunden sind,
- Algorithmen zur Signalverarbeitung von Zeitreihen, die von Sensoren oder (Teil-)Bilddaten-Messungen erstellt wurden, die direkt oder indirekt verschiedene Bewegungsmerkmale eines Patienten (Herzschlag, Atemzyklus, Darmbewegung, Körperbewegung) messen und bestimmen, mit welcher Wahrscheinlichkeit die analysierten Zeitreihen mit Bildqualitätsproblemen korrelieren,
- Klassifikationsalgorithmen, die eine Anzahl von Bildqualitätsbewertungsklassen bereitstellen.
- Regressionsalgorithmen, die eine kontinuierliche Bewertung der Bildqualität innerhalb eines endlichen Wertebereichs liefern,
- IQA-Algorithmen zur Qualitätsbeurteilung von unbearbeiteten 2D oder 3D K-Raum-Bildern oder räumlichen 2D- oder 3D-Bildern,
- IQA-Algorithmen zur Qualitätsbeurteilung für eine zeitaufgelöste Bildgebung.

Es ist vorteilhaft, wenn ein IQA-Algorithmus konfigurierbare Parameter aufweist, die es dem Benutzer des bildgebenden Systems ermöglichen, die Empfindlichkeit (bzw. Spezifität) des IQA-Algorithmus oder von dessen Ergebnissen anzupassen, um eine unzureichende Bildqualität gemäß den Vorlieben des Benutzers zu erkennen. Ein Beispiel wäre ein IQA-Algorithmus in Form eines als Regressionsmodell trainierten Deep-Learning-Netzwerks, das ein diagnostisches Qualitätsmaß durch Schwellwertbildung eines vorhergesagten Bildqualitätswerts liefert (wobei z. B. 0 der schlechteste und 1 der bestmögliche Wert sein kann). Die Erkennungsempfindlichkeit eines schlechten Bildes kann angepasst werden, indem der Schwellwert zur Entscheidung "schlecht" erhöht oder gesenkt wird.

Es ist bevorzugt, dass zumindest ein Teil der IQA-Algorithmen Informationen zur Anwendbarkeit aufweist und/oder mittels der Änderung von Werten vorgegebener konfigurierbarer Parameter konfiguriert werden kann, bevorzugt die Sensitivität und/oder Spezifität der jeweiligen IQA-Algorithmen. Es ist dabei nicht unbedingt notwendig, die IQA-Algorithmen selber anzupassen, was besonders bei trainierten lernfähigen Netzwerken schwierig sein könnte, sondern einfach die Weiterverarbeitung deren Ergebnisse konfigurierbar zu gestalten. Beispielsweise kann ein IQA-Algorithmus einen numerischen Qualitätswert liefern, der durch einen Vergleich mit einem vorbestimmten, konfigurierbaren Wertebereich verändert oder ausgewertet wird.

Wie oben angedeutet, muss die IQA-Einheit nicht unbedingt aus einer Anzahl von IQA-Algorithmen bestehen, sondern sie kann noch weitere Komponenten aufweisen. Diese Komponenten können der Verarbeitungsreihenfolge durch mehrere IQA-Algorithmen dienen (nachfolgend genauer ausgeführt) oder die Ergebnisse von einer Anzahl von IQA-Algorithmen weiterverarbeiten.

Eine bevorzugte Komponente der IQA-Einheit ist eine Aggregations-Einheit, welche für eine Aggregation von Daten ausgelegt ist. Ein bevorzugter Fall für eine Aggregation ist die Ansammlung mehrerer räumlicher 2D-Bilder zur Formung eines räumlichen 3D-Bilds. Dies kann vor der Eingabe der Bilddaten in einen IQA-Algorithmus erfolgen, z. B. wenn dieser nur mit 3D-Daten arbeiten kann, oder bei der Auswertung dessen Ergebnissen (z. B. weil er nur eine Auswertung von 2D-Bildern liefert aber ein Qualitätsmaß für ein 3D-Bild benötigt wird. Ein weiterer bevorzugter Fall sind Steuerprotokolle, mit denen mehrere 2D-räumliche+1D-zeitliche DICOM-Daten erzeugt werden. Je nachdem welche Dimensionen der IQA-Algorithmus prozessieren kann, müssten seine Ergebnisse ebenfalls aggregiert werden. Hier könnte die IQA-Einheit mit einem 2D-räumlich basierten IQA-Algorithmus so gestaltet sein, dass sie durch Aggregation in der Zeitdimension ein Qualitätsmaß für das 2D-räumliche+1D-zeitliche DICOM generieren kann, so dass ein Benutzer ein Qualitätsmaß für die gesamte DICOM-Datei erhält. Die Abkürzung DICOM steht dabei für "Digital Imaging and Communications in Medicine" ein Standard in der medizintechnischen Bildgebung.

Ein bevorzugtes Ziel der Erfindung ist, dass ein Benutzer für jede eingegebene DICOM-Datei ein Qualitätsmaß für die gesamte Datei ermittelt. Daher sollte im Allgemeinen zur Ermittlung des Qualitätsmaßes für p-dimensionale Bilddaten bei der Nutzung eines IQA-Algorithmus mit der Input-Dimensionalität q eine Aggregation über die p-q Dimensionen stattfinden, sofern p>q. Bei p=q ist eine Aggregation nicht nötig. Bei p<q ist der IQA-Algorithmus auf die Input-Daten nicht oder nur teilweise anwendbar.

Wenn die Spezifikationen für die Eingabedimension für einen IQA-Algorithmus (z. B. räumlich 2D) nicht mit der Dimension der erfassten Bilddaten (z. B. räumlich 3D) übereinstimmt, sollte die IQA-Einheit also eine Aggregationsoption bzw. besagte Aggregations-Einheit aufweisen, um effektiv ein Qualitätsmaß (z. B. für eine DICOM-Serie) zu erstellen.

Die IQA-Einheit kann aber auch eine Aggregations-Einheit aufweisen, die dazu ausgestaltet ist, Ergebnisse von IQA-Algorithmen oder Scores bzw. Qualitätsmaße zusammenzufassen und insbesondere auch zu bearbeiten, z. B. durch Bildung eines Mittelwerts, Medians oder des Maximums oder Minimums.

Eine weitere bevorzugte Komponente der IQA-Einheit ist eine Scoring-Einheit, die dazu ausgelegt ist, aus einem Ergebnis eines IQA-Algorithmus ein Qualitätsmaß zu generieren. Zwar liefern viele IQA-Algorithmen bereits verwendbare Qualitätswerte, jedoch könnte der Fall vorliegen, dass ein anderer Wertebereich benötigt wird (z. B. 0% bis 100% statt 0 bis 10) oder die Ergebnisse mehrerer IQA-Algorithmen zusammengefasst werden müssen oder Ergebnisse zuerst aggregiert wurden. Die Scoring-Einheit ist dabei idealer Weise so gestaltet, dass sie das gewünschte Format für das Qualitätsmaß ausgibt.

Eine weitere bevorzugte Komponente der IQA-Einheit ist eine Rating-Einheit, die dazu ausgelegt ist, aus einem Ergebnis eines IQA-Algorithmus oder aus einem Qualitätswert einer Scoring-Einheit ein Ergebnis zu generieren, welches als (ggf. zusätzliches) Qualitätsmaß ausgegeben werden kann. Dieses Rating kann z. B. die Bezeichnungen "gut" und "schlecht" umfassen, oder einer Auswahl an Zahlen, z. B. den Noten 1 bis 6). Auch kann ein Rating bereits eine Warnung umfassen, z. B. die Nachricht "schlechte Aufnahme" oder Steuerbefehle zur Ausgabe einer Warnung durch das bildgebende System. Die Rating-Einheit könne sogar im Falle einer schlechten Qualität Informationen generieren, die Aufschluss darüber geben, welche Parameter geändert werden sollten, z. B. die Nachricht "Achtung, Aufnahme nicht genügend Kontrast". Es kann auch einfach aufgrund eines binären Ratings (bei dem Befund "schlecht") die aktuelle Aufnahme gestoppt werden.

Hier sollte beachtet werden, dass die Aggregation, ein Scoring oder ein Rating bereits von einem IQA-Algorithmus durchgeführt werden kann, dass also die vorgenannten Komponenten Teil eines IQA-Algorithmus sind. Es ist aber auch, insbesondere bei ändernden Konfigurationen von IQA-Algorithmen, von Vorteil, wenn die IQA-Algorithmen eigenständige (Software- )Komponenten sind und eine Aggregation und/oder ein Scoring und/oder eine Klassifikation von zusätzlichen Komponenten durchgeführt wird.

Eine bevorzugte Steuereinrichtung umfasst eine Konfigurations-Einheit. Diese Konfigurationseinheit dient der Konfiguration der IQA-Einheit und ist zum Empfang und/oder zum Generieren von Konfigurations-Werten und der Einstellung der IQA-Einheit, insbesondere eines IQA-Algorithmus oder anderer Komponenten zur Berechnung des Qualitätsmaßes, mit den Konfigurations-Werten ausgelegt.

Es ist dabei bevorzugt, dass die Konfigurations-Einheit und die IQA-Einheit dazu ausgestaltet sind, dass eine Anzahl der eingesetzten IQA-Algorithmen basierend auf einer Benutzereingabe und/oder basierend auf einem bei der Aufnahme der Bilddaten verwendeten Steuerprotokoll konfiguriert oder zusammengestellt wird und/oder dass die Weiterverarbeitung von Ergebnissen einer Anzahl von IQA-Algorithmen konfiguriert wird.

Die Konfigurationseinheit und die IQA-Einheit arbeiten also bevorzugt sehr eng zusammen, wobei die Konfigurationseinheit durchaus eine Komponente der IQA-Einheit sein kann. Die IQA-Einheit muss dabei konfigurierbar sein was bedeutet, dass die Arbeit ihrer Komponenten und/oder das Zusammenspiel der Komponenten auf Parametern beruht, deren Werte geändert werden können. Die Art und der Grad der Konfiguration kann dabei von einer Benutzereingabe stammen (im Folgenden noch ausführlicher erklärt) oder aus einem für die Bildaufnahme verwendeten Steuerprotokoll abgeleitet werden.

Bevorzugt wird eine Anzahl der eingesetzten IQA-Algorithmen konfiguriert. Damit ist nicht unbedingt nur gemeint, dass die Zahl der IQA-Algorithmen geändert wird (z. B. zwei statt einem), sondern auch, dass konfigurierbar ist, welche IQA-Algorithmen überhaupt verwendet werden sollen. Beispielsweise kann durch einen Benutzer vorgegeben oder durch ein Steuerprotokoll impliziert werden, dass ein bestimmter Regressionsalgorithmus und ein bestimmter Klassifikationsalgorithmus in Kombination verwendet werden sollen. Es kann bei einem anderen Steuerprotokoll aber stattdessen ein besonders trainierter Deep-Learning Algorithmus eingesetzt werden.

Es kann aber auch die Art (also welche Komponente oder die Verarbeitung welcher Daten genau konfiguriert wird) oder der Grad (wie stark eine Änderung der bisherigen Parameterwerte sein soll bzw. ein absoluter Wert für die Konfiguration) vorgegeben werden. Beispielsweise kann die Sensitivität oder die Spezifität der IQA-Einheit oder einer ihrer Komponenten basierend auf einem aktuell applizierten Steuerprotokoll angepasst werden, z. B. abhängig von Scan-Indikation und/oder abhängig von den interessierenden diagnostischen Regionen. Die Konfiguration der IQA-Einheit kann dabei gemäß Auswahldaten gesteuert werden, die angeben, welche Komponente der IQA-Einheit konfiguriert werden soll.

Bei IQA-Algorithmen, die keine Konfigurationsparameter aufweisen (dies ist z. B. bei vielen Deep-Learning Algorithmen der Fall), ist bevorzugt, dass die konfigurierbaren Parameter nur auf den Input und/oder Output eines solchen IQA-Algorithmus wirken, nämlich insbesondere auf das Thresholding und/oder die Klassifikation des Outputs und ggf. der Art der Aggregation, z. B. 2D-Bild-spezifische Ergebnisse zu einem 3D-spezifischen Qualitätsmaß.

Für eine Konfiguration durch einen Benutzer ist eine spezielle (grafische) Konfigurationsoberfläche von Vorteil. Eine bevorzugte Steuereinrichtung umfasst daher eine Konfigurations-Benutzerschnittstelle, über die ein Benutzer Konfigurations-Werte für die Konfigurations-Einheit generieren und/oder in diese eingeben kann und/oder zur Generierung des Qualitätsmaßes eine Anzahl von IQA-Algorithmen aus einer Mehrzahl von IQA-Algorithmen auswählen kann, insbesondere individuell für eine Anzahl von Steuerprotokollen.

Die Eingabe von Konfigurations-Werten kann einfach mittels der Auswahl des entsprechenden Parameters und einer Eingabe eines Konfigurations-Wertes erfolgen. Es könnte aber auch auf einer grafischen Oberfläche ein Einstellelement angewählt und verändert werden und diese Veränderung dann den entsprechenden Konfigurations-Wert darstellen.

Die Konfiguration kann auch automatisch erfolgen je nach Daten die aus anderen Datenquellen (z. B. RIS) vorliegen und indikationsgetrieben oder patientenspezifisch sein können.

Was die Auswahl von IQA-Algorithmen betrifft, kann dem Benutzer eine Liste von Verfügbaren IQA-Algorithmen zur Verfügung gestellt werden, aus denen dieser die favorisierten IQA-Algorithmen auswählen kann. Hierzu sollte jedoch beachtet werden, dass nicht alle IQA-Algorithmen für alle Bilddaten gleich gut (oder überhaupt) geeignet sind. Es ist daher bevorzugt, diese Auswahl vom System automatisiert zu beeinflussen.

Es ist diesbezüglich bevorzugt, dass dem Benutzer über die Konfigurations-Benutzerschnittstelle für den Einsatz mit einem Steuerprotokoll eine spezifische Anzahl von möglichen anwählbaren IQA-Algorithmen anzeigt wird, die nach oder während der Applikation dieses Steuerprotokolls für die Ermittlung des Qualitätsmaßes verwendet werden sollen, bevorzugt wobei dazu eine automatisierte Prüfung der Kompatibilität des IQA-Algorithmus mit dem angewandten Steuerprotokoll durchgeführt wird, welche die Spezifikationen des Steuerprotokolls (z. B. Sequenz, Rekonstruktion, Protokollparametersatz) mit vorgegebenen Spezifikationen für die Anwendbarkeit der IQA-Algorithmen abgleicht und bevorzugt nur kompatible IQA-Algorithmen anzeigt und/oder wenn die Prüfung der Kompatibilität eine Inkompatibilität eines IQA-Algorithmus mit einem bestimmten Steuerprotokoll anzeigt, einem Benutzer Rückmeldung gibt, welche minimalen Protokollmodifikationen zu einer Kompatibilität mit welchem zusätzlichen IQA-Algorithmus führen würden. Die IQA-Algorithmen sollten dazu Spezifikationen für die Anwendbarkeit aufweisen, die von einer automatisierten Prüfung der Kompatibilität der IQA-Algorithmen verwendet werden können. Im Falle von MRT-Aufnahmen könnte eine solche Spezifikation "Nur für T2-Kontrast" lauten. Für Bilddaten mit anderen Kontrasten, könnte dann ein Anwählen der betreffenden IQA-Algorithmen gesperrt sein.

In der Praxis könnte zum Zeitpunkt der Konfiguration und/oder des Starts einer Aufnahme eine automatische Prüfung der Kompatibilität der IQA-Algorithmen ausgeführt werden, um deren Kompatibilität mit jedem Scanschritt zu bewerten. Zu diesem Zweck kann eine automatisierte Prüfung der Kompatibilität eines IQAA mit dem Steuerprotokoll die Spezifikationen des angegebenen Scanschritts (z. B. Rekonstruktion, Sequenz, Protokollparametersatz) mit Spezifikationen für die Anwendbarkeit der QA-Algorithmen vergleichen. Insbesondere zum Zeitpunkt des Programmstarts kann eine Prüfung stattfinden, ob die IQA-Algorithmen geeignet sind oder ob ggfs. noch Sensoren angeschlossen werden müssen (z. B. eine Kopfspule mit Beatsensor oder ein EKG).

Wenn die Kompatibilitätsprüfung des IQAA-Protokolls eine Inkompatibilität mit einem bestimmten Scanschritt aufweist, könnte dies z. B.
- nur kompatible IQAAs anzeigen, die für diesen Scanschritt konfiguriert werden sollen und/oder
- dem Benutzer des Scanners Rückmeldung geben, welche minimalen Protokollmodifikationen zu einer Kompatibilität mit welchen zusätzlichen IQAAs führen würden.

Wenn für einen Scanschritt eine oder mehrere IQA-Algorithmen konfiguriert sind, ist bevorzugt, dass die betreffenden IQA-Algorithmen automatisch direkt nach der Datenerfassung dieses Scanschritts ausgeführt werden.

Abhängig von der Art des IQAA können zusätzliche Konfigurationseinstellungen vorhanden sein, z. B. eine Option zum Anpassen der Empfindlichkeit von QA-Algorithmen, um ein Bildqualitätsproblem zu erkennen (z. B. niedrige oder mittlere oder hohe Empfindlichkeit oder ein kontinuierlicher Schwellenwert innerhalb einer bestimmten Skala, z. B. 0,0 auf 1,0).

Außerdem können IQA-Algorithmen Konfigurationsoptionen aufweisen, die bestimmen, wie IQA-Algorithmen, die sich auf n-dimensionale Eingabedaten stützen, aggregiert werden können, um eine Qualitätsbewertung für eine m-dimensionale Bildserie zu erhalten. Beispielsweise kann ein IQA-Algorithmus, der in der Lage ist, Qualitätsbewertungen für einzelne Bildframes (z. B. räumliche 2D-Bilder) zu generieren, einen Konfigurationsparameter anbieten, wie die Bewertungen der 2D-Frames eines Bildes aggregiert werden sollen, um eine Qualitätsbewertung für die vollständige Bildserie zu generieren (z. B. ein räumliches 3D-Bild). Verfügbare Score-Aggregationsoptionen können die Berechnung des Durchschnitts, Medians, Minimums oder Maximums von Qualitätsmaßen von Bildframes innerhalb einer Bildserie beinhalten.

Gemäß einer bevorzugten Steuereinrichtung ist diese, insbesondere deren IQA-Einheit oder eine Anzahl von IQA-Algorithmen der IQA-Einheit oder die Konfigurationseinheit, dazu ausgelegt, mindestens einen Konfigurations-Wert basierend auf einem aktuell applizierten Steuerprotokoll (automatisch) zu ändern. Es ist dabei bevorzugt, dass eine Detektionsschwelle der IQA-Einheit, insbesondere eines IQA-Algorithmus der IQA-Einheit, eingestellt wird und/oder basierend auf einem aktuell applizierten Steuerprotokoll eine Anzahl von IQA-Algorithmen ausgewählt wird, die in der IQA-Einheit zur Ermittlung des Qualitätsmaßes verwendet werden sollen.

Es sollte beachtet werden, dass auf medizintechnischen Bildern (oder Daten im Allgemeinen) bestimmte Datenpunkte für das Lesen sehr relevant sind, während andere Datenpunkte weniger wichtig sind. Beispielsweise sind in räumlichen 2D- oder 3D-Bildern die Hintergrundpixel normalerweise nicht von hoher Relevanz (außer für die Bewertung bestimmter Artefakte, z. B. Bewegung oder Signal-Rausch-Verhältnis oder der Präsenz von Bewegungsartefakten), während z. B. in einem neurologischen Scan weiße und graue Substanz von hoher Relevanz sind. Dementsprechend ist es von Vorteil, die Signifikanz eines einzelnen Datenpunkts für die Erzeugung eines Qualitätsmaßes zu gewichten.

Gemäß einer bevorzugten Steuereinrichtung ist diese, insbesondere die IQA-Einheit oder die Konfigurationseinheit, dazu ausgelegt, basierend auf einem aktuell applizierten Steuerprotokoll die Relevanz von Datenpunkten in den Bilddaten für eine mit diesem Steuerprotokoll verbundene medizintechnische Untersuchung in einer Relevanzkarte (numerisch) zu erfassen, insbesondere Datenpunkte zur Darstellung von Organen oder Körperbereichen. Es ist dabei bevorzugt, dass basierend auf dieser Relevanzkarte ein räumlich gewichtetes Qualitätsmaß erstellt wird, bevorzugt wobei Datenpunkte, die für eine vorgegebene Untersuchung weniger relevant sind, mit einer geringeren Gewichtung in dem Qualitätsmaß berücksichtigt werden als Datenpunkte, die für die vorgegebene Untersuchung relevanter sind. Dies kann z. B. dadurch realisiert werden, dass Rand- oder Hintergrundpixel weniger gewichtet werden als Pixel von den interessierenden anatomischen Zielstrukturen. Bevorzugt kann ein Steuerprotokoll oder eine Scan-Indikation auf wohldefinierte interessierende Regionen (ROI) oder anatomische Gewebe hinweisen, die für die jeweilige diagnostische Fragestellung von Hauptinteresse sind, z. B. erfordert ein Augen-Scan-Programm gute Bildqualität für die Augen und Sehnerven. In dieser bevorzugten, fortgeschrittenen Variante sollte die IQA-Einheit zur Generierung des Qualitätsmaßes die Daten von einer diagnostischen Zielregion stärker gewichten als andere Daten. Dazu ist bevorzugt, dass die IQA-Einheit eine Form von direktem oder indirektem anatomischem Verständnis besitzt, z. B. durch Verwendung von Orientierungspunkterkennung, Objekterkennung, Atlas-basiert Lokalisierungsfähigkeiten oder segmentierungsbasierte Lokalisierungsfähigkeiten oder jede Kombination der erwähnten oder ähnlichen Methoden. Bevorzugt können dazu IQA-Algorithmen der IQA-Einheit sowohl eine anatomiespezifische als auch eine indikationsspezifische Bildqualitätsbeurteilung besitzen, um die Anforderungen eines Radiologen für das Lesen von Bildern vollständig zu modellieren. In einer weiteren bevorzugten Variante der IQA-Algorithmen könnten Patienten-Compliance, zustandsspezifische Besonderheiten (z. B. Demenz, Parkinson-Krankheit, Fötus) oder situationsbezogene Zustände (z. B. zeitkritischer Notfall) von den IQA-Algorithmen berücksichtigt werden, um die Erkennungs-/Akzeptanzschwelle fallspezifisch anzupassen. Eine Anpassung kann auch indikationsgetrieben sein, was insbesondere über eine vorbestimmte Strategie abgebildet werden kann.

Gemäß einer bevorzugten Steuereinrichtung umfasst die IQA-Einheit eine Mehrzahl von IQA-Algorithmen, und ist dazu ausgelegt oder dazu einstellbar, dass basierend auf zwei oder mehr IQA-Algorithmen ein Qualitätsmaß für die Qualität der Daten einer einzigen Bildaufnahme ermitteln. Es ist dabei bevorzugt, dass
- in einer parallelen Konfiguration eine Mehrzahl von IQA-Algorithmen ein individuelles Ergebnis aus den Bilddaten generieren und basierend auf diesen Ergebnissen ein zusammenfassendes Qualitätsmaß ermittelt wird, bevorzugt ein ausgeglichener oder gewichteter Durchschnitt, ein Medianwert, ein Minimum oder ein Maximum der Ergebnisse,
- in einer seriellen Konfiguration zunächst ein erster IQA-Algorithmus ein Ergebnis generiert und in dem Fall, dass sich das Ergebnis in einem vorbestimmten Unsicherheitsbereich befindet, ein weiterer IQA-Algorithmus ein weiteres Ergebnis generiert und aus dem Ergebnis des letzten IQA-Algorithmus oder basierend auf mehreren der Ergebnisse ein Qualitätsmaß ermittelt wird,
- eine Untergruppe der IQA-Algorithmen parallel konfiguriert ist und mit einer Anzahl von anderen IQA-Algorithmen oder Untergruppen von IQA-Algorithmen seriell konfiguriert ist.

Diesbezüglich ist bevorzugt, dass die Einstellung, welche IQA-Algorithmen in paralleler Konfiguration und welche in serieller Konfiguration ausgeführt werden, bestimmt wird durch:
- eine Voreinstellung des klinischen Bedieners,
- eine Priorität eines Bildqualitätsproblems, auf das ein bestimmter IQA-Algorithmus der IQA-Einheit abzielt (z. B. zuerst hohe Priorität),
- eine Häufigkeit des Auftretens eines Bildqualitätsproblems im klinischen Zentrum (z. B. häufiges Auftreten zuerst),
- verfügbare Rechenressourcen (z. B. mehr IQA-Algorithmen parallel, wenn mehr Ressourcen verfügbar sind),
- Art und Ressourcenverbrauch der (konfigurierten) IQA-Algorithmen (z. B. relevant für die-Gruppierung),
- technische Notwendigkeiten (z. B. kann eine Bildanalytik erst stattfinden, wenn ein Bild da ist und eine Sensoranalytik nur wenn Signale da sind).

Bevorzugt würde bei einer teil- oder vollsequentiellen Bewertung durch IQAAs ein IQAA (oder eine Untergruppe von IQAAs) die Bildqualität nur dann bewerten, wenn der vorangegangene IQAA (oder eine Untergruppe von IQAAs) eine minimale Bildqualität ermittelt hat. Mit diesem Ansatz konnten die Auswertungszeit, die Rechenressourcen und vor allem die Zeit bis zur Auslösung von Maßnahmen optimiert werden. Als Beispiel: Wenn gravierende Artefakte von einem ersten IQAA festgestellt werden, ist es möglicherweise nicht erforderlich, eine zusätzliche Bewertung mittels weiteren IQAAs durchzuführen, da der erste IQAA die aufgenommenen Bilder bereits als undiagnostisch bewertet hat. Somit kann eine zusätzliche Auswertungszeit eingespart und eine Abhilfemaßnahme (z. B. Wiederholung des letzten Scanschritts) direkt ausgeführt werden.

In der Radiologie hängt die für die diagnostische Befundung erforderliche Bildqualität häufig von der Scan-Indikation ab, d.h. von der diagnostisch relevanten Merkmalsart und Strukturgröße. Bevorzugt passt daher eine vorteilhafte IQA-Einheit eine Detektionsschwelle automatisch abhängig von der Scan-Indikation an und/oder ermöglicht es dem Benutzer, indikationsspezifische Detektionsschwellen für IQA-Algorithmen oder eine Verarbeitung von Ergebnissen von IQA-Algorithmen einzustellen. Beispielsweise erfordert die Diagnose von Multipler Sklerose im Gehirn eine viel höhere Bildschärfe und Auflösung als die Bestätigung/Widerlegung eines großen Tumors. Daher sollte bei einem Scan mit Indikation MS die Nachweisschwelle sensitiver eingestellt werden als bei einem großen Tumorscan.

Bevorzugt werden einem Benutzer Ergebnisse einer Anzahl von verwendeten IQA-Algorithmen in einer Scan-Benutzeroberfläche angezeigt, um den Benutzer des bildgebenden Systems während oder direkt nach der Erfassung und Bewertung durch die IQA-Algorithmen über die Qualität der erfassten Bilder zu informieren. Wenn ein Bildqualitätsproblem erkannt wird, kann der Benutzer sofort Korrekturmaßnahmen ergreifen.

Die IQA-Algorithmen sind bevorzugt unabhängige Module, die so konfiguriert werden können, dass sie automatisch bei jedem Bilderfassungsschritt in einer MR-Untersuchung ausgeführt werden, wie vom Benutzer bevorzugt.

Gemäß einem bevorzugten Verfahren sind die Bilddaten Daten einer DICOM-Serie und/oder MR-Spektroskopiemessungen, quantitative T1- oder T2-Karten oder MR-Fingerprinting-Karten. Dabei wird bevorzugt das Qualitätsmaß für die gesamte DICOM-Serie, ein 3D-Bild, 3D Rohdaten, ein 2D-Bild, 2D-Rohdaten, eine Anzahl von Schichten in einem Stack, ein Segment eines Bildes oder ein K-Raum-Segment generiert.

Gemäß einem bevorzugten Verfahren wird in dem Fall, dass eine Eingabedimensionsspezifikation der IQA-Einheit nicht mit der Dimension der erfassten Bilddaten übereinstimmt, eine Aggregation der Daten durchgeführt. Es ist dabei bevorzugt, dass die IQA-Einheit einen Konfigurationsparameter aufweist, der angibt, wie die Aggregation durchgeführt werden soll und dieser Konfigurationsparameter über eine Konfigurations-Benutzerschnittstelle konfiguriert werden kann. Dabei beinhaltet die IQA-Einheit bevorzugt eine Auswahl von Score-Aggregationsoptionen der Gruppe Berechnung des Durchschnitts, Berechnung des Medians, Berechnung des Minimums und Berechnung des Maximums von Qualitätsmaßen von Teilbildern und/oder Ergebnissen von IQA-Algorithmen. Die Berechnung sollte im Rahmen der Konfiguration ausgewählt werden können. Es wird also bei mehreren Ergebnissen von verwendeten IQA-Algorithmen bzw. mehreren Teilbildern (ggf. gewichtet) ein Durchschnittswert oder ein Median oder ein Minimum bzw. ein Maximum berechnet.

Steht genügend Rechenleistung zur Verfügung, ist es bevorzugt, die Erfindung lokal in einer Hardware des medizintechnischen bildgebenden Systems, z. B. einer Scanner-Hardware, oder einer anderen, direkt mit dem Scanner verbundenen Hardware zu integrieren, um die Auswertungszeit durch Wegfall von Netzwerk-Latenzen so kurz wie möglich zu halten.

Komponenten der Erfindung können aber auch als ein "Cloud-Dienst" vorliegen. Ein solcher Cloud-Dienst dient der Bearbeitung von Daten, insbesondere mittels einer künstlichen Intelligenz, kann aber auch ein Dienst basierend auf herkömmlichen Algorithmen sein oder ein Dienst, bei dem im Hintergrund eine Auswertung durch Menschen stattfindet. Generell ist ein Cloud-Dienst (im Folgenden auch kurz als "Cloud" bezeichnet) eine IT-Infrastruktur, bei der über ein Netzwerk z. B. Speicherplatz oder Rechenleistung und/oder eine Anwendungssoftware zur Verfügung gestellt wird. Die Kommunikation zwischen dem Anwender und der Cloud erfolgt dabei mittels Datenschnittstellen und/oder Datenübertragungsprotokollen. Im hier vorliegenden Fall ist besonders bevorzugt, dass der Cloud-Dienst sowohl Rechenleistung als auch Anwendungssoftware zur Verfügung stellt.

Im Rahmen eines bevorzugten Verfahrens erfolgt eine Bereitstellung von Bilddaten über das Netzwerk an den Cloud-Dienst. Dieser umfasst ein Rechensystem, z. B. einen Computercluster, der das erfindungsgemäße Verfahren ausführen kann (insbesondere eine IQA-Einheit als Softwaremodul) und das in der Regel nicht den lokalen Rechner des Benutzers umfasst. Diese Cloud kann insbesondere durch die medizintechnische Einrichtung, die auch die medizintechnischen Systeme bereitstellt, zur Verfügung gestellt werden. Beispielsweise werden die Bilddaten über ein RIS (Radiologieinformationssystem) oder PACS an ein (Remote-) Rechnersystem (die Cloud) gesendet. Bevorzugt stellen das Rechensystem der Cloud, das Netzwerk sowie das medizintechnische System einen Verbund im datentechnischen Sinne dar. Das Verfahren kann dabei mittels einer Befehls-konstellation in dem Netzwerk realisiert werden. Die in der Cloud berechneten Daten ("Ergebnisdaten") werden später wieder über das Netzwerk zu dem lokalen Rechner des Anwenders gesendet.

Gemäß einer bevorzugten Steuereinrichtung ist die IQA-Einheit ein Software-Modul auf einer Verarbeitungshardware der Scannerhardware, auf einem Peripherie-Gerät eines medizintechnischen bildgebenden Systems, insbesondere eines MRT-Systems, eines CT-Systems oder eines PET-Systems, oder in einer Cloud.

Bevorzugt ist eine Konfigurations-Benutzerschnittstelle als Software-Modul in einer grafischen Scanner-Benutzerschnittstelle integriert.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine schematische Darstellung eines Magnetresonanztomographiesystems gemäß einem Ausführungsbeispiel der Erfindung,
Figur 2 ein Ausführungsbeispiel für eine erfindungsgemäße IQA-Einheit und eine Konfigurationseinheit,
Figur 3 die Funktionsweise einer IQA-Einheit,
Figur 4 eine serielle Kombination von IQA-Algorithmen,
Figur 5 eine parallele Kombination von IQA-Algorithmen,
Figur 6 ein Blockdiagramm eines Ausführungsbeispiels eines erfindungsgemäßen Verfahrens,
Figur 7 eine räumliche Priorisierung von Bereichen.

In den folgenden Figuren sind nur für die Erfindung wesentliche oder zu ihrem Verständnis hilfreiche Elemente eingezeichnet. So sind z. B. keine Schichtselektionsgradienten dargestellt, obwohl sie in der Pulssequenz durchaus vorhanden sein können.

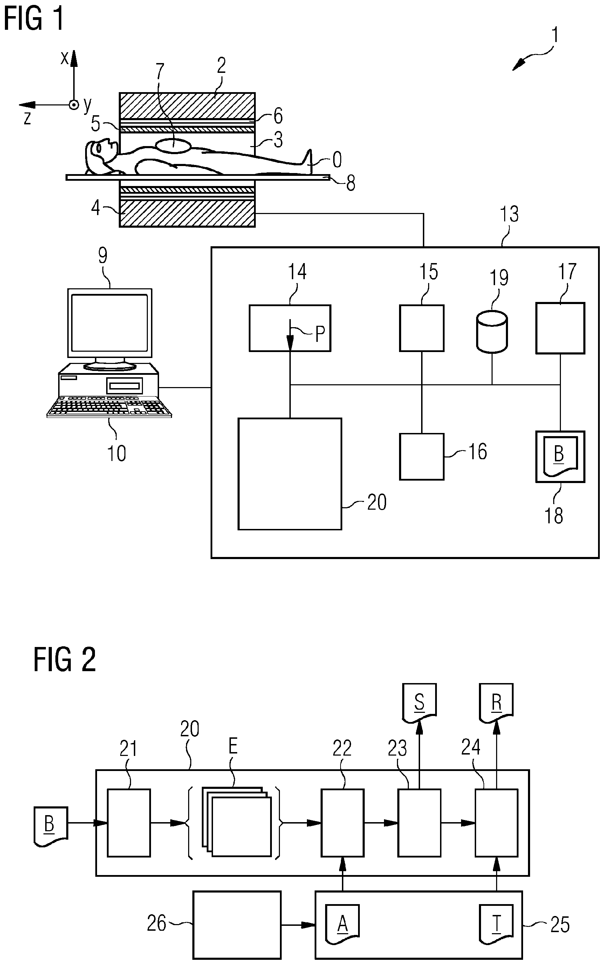

In Figur 1 ist grob schematisch ein Magnetresonanztomographiesystem 1 als Beispiel für ein medizintechnisches bildgebendes System 1 dargestellt. Es umfasst zum einen den eigentlichen Magnetresonanzscanner 2 mit einem Untersuchungsraum 3 bzw. Patiententunnel, in den auf einer Liege 8 ein Patient oder Proband positioniert ist, in dessen Körper sich das eigentliche Untersuchungsobjekt O befindet. Auch wenn in dem dargestellten Beispiel das Untersuchungsobjekt O im Torso abgebildet ist, wird die Diffusions-Tensor-Bildgebung auch oft für Aufnahmen des Gehirns verwendet, da sie besonders gut zur Abbildung neurologischer Strukturen geeignet ist.

Der Magnetresonanzscanner 2 ist in üblicher Weise mit einem Grundfeldmagnetsystem 4, einem Gradientensystem 6 sowie einem HF-Sendeantennensystem 5 und einem HF-Empfangsantennen-system 7 ausgestattet. In dem dargestellten Ausführungsbeispiel handelt es sich bei dem HF-Sendeantennensystem 5 um eine im Magnetresonanzscanner 2 fest eingebaute Ganzkörperspule, wogegen das HF-Empfangsantennensystem 7 aus am Patienten bzw. Probanden anzuordnenden Lokalspulen besteht (in der Figur nur durch eine einzelne Lokalspule symbolisiert). Grundsätzlich können aber auch die Ganzkörperspule als HF-Empfangsantennensystem und die Lokalspulen als HF-Sendeantennensystem genutzt werden, sofern diese Spulen jeweils in unterschiedliche Betriebsweisen umschaltbar sind. Das Grundfeldmagnetsystem 4 ist hier in üblicher Weise so ausgebildet, dass es ein Grundmagnetfeld in Längsrichtung des Patienten erzeugt, d. h. entlang der in z-Richtung verlaufenden Längsachse des Magnetresonanzscanners 2. Das Gradientensystem 6 umfasst in üblicher Weise einzeln ansteuerbare Gradientenspulen, um unabhängig voneinander Gradienten in x-, y- oder z-Richtung schalten zu können. Zudem enthält der Magnetresonanzscanner 2 (nicht dargestellte) Shimspulen, die in üblicher Weise ausgebildet sein können.

Das dargestellte Magnetresonanztomographie-System ist eine Ganzkörperanlage mit einem Patiententunnel, in den ein Patient komplett eingebracht werden kann. Grundsätzlich kann die Erfindung aber auch an anderen Magnetresonanztomographie-Systemen, z. B. mit seitlich offenem, C-förmigen Gehäuse, verwendet werden.

Das Magnetresonanztomographie-System 1 weist weiterhin eine zentrale Steuereinrichtung 13 auf, die zur Steuerung des MR-Systems 1 verwendet wird. Diese zentrale Steuereinrichtung 13 umfasst eine Sequenzsteuereinheit 14. Mit dieser wird die Abfolge von Hochfrequenz-Pulsen (HF-Pulsen) und von Gradientenpulsen in Abhängigkeit von einem gewählten Steuerprotokoll P zur Aufnahme mehrerer Schichten in einem interessierenden Volumenbereich des Untersuchungsobjekts innerhalb einer Messsitzung gesteuert. Üblicherweise sind verschiedene Steuerprotokolle P für unterschiedliche Messungen in einem Speicher 19 hinterlegt und können von einem Bediener ausgewählt (und bei Bedarf gegebenenfalls geändert) und dann zur Durchführung der Messung genutzt werden.

Zur Ausgabe der einzelnen HF-Pulse einer Pulssequenz eines Steuerprotokolls P weist die zentrale Steuereinrichtung 13 eine Hochfrequenzsendeeinrichtung 15 auf, die die HF-Pulse erzeugt, verstärkt und über eine geeignete Schnittstelle (nicht im Detail dargestellt) in das HF-Sendeantennensystem 5 einspeist. Zur Steuerung der Gradientenspulen des Gradientensystems 6, um entsprechend dem vorgegebenen Steuerprotokoll P die Gradientenpulse passend zu schalten, weist die Steuereinrichtung 13 eine Gradientensystemschnittstelle 16 auf. Über diese Gradientensystemschnittstelle 16 könnten die Gradientenpulse appliziert werden. Die Sequenzsteuereinheit 14 kommuniziert in geeigneter Weise mit der Hochfrequenzsendeeinrichtung 15 und der Gradientensystemschnittstelle 16 zur Ausführung des Steuerprotokolls P.

Die Steuereinrichtung 13 weist außerdem eine (ebenfalls in geeigneter Weise mit der Sequenzsteuereinheit 14 kommunizierende) Hochfrequenzempfangseinrichtung 17 auf, um innerhalb der durch das Steuerprotokoll P vorgegebenen Auslesefenster koordiniert mittels des HF-Empfangsantennensystems 7 Magnetresonanz-Signale zu empfangen und so die Rohdaten zu akquirieren.

Eine Rekonstruktionseinheit 18 übernimmt hier die akquirierten Rohdaten und rekonstruiert daraus Bilddaten B. Auch diese Rekonstruktion erfolgt in der Regel auf Basis von Parametern, die in dem jeweiligen Steuerprotokoll P vorgegeben sein können. Diese Bilddaten können dann beispielsweise in einem Speicher 19 hinterlegt werden.

Wie im Detail durch ein Einstrahlen von HF-Pulsen und das Schalten von Gradientenpulsen geeignete Rohdaten akquiriert und daraus MR-Bilder oder Parameter-Karten rekonstruiert werden können, ist dem Fachmann grundsätzlich bekannt und wird daher hier nicht näher erläutert.

Die Steuereinrichtung 13 umfasst eine IQA-Einheit 20, welche die Qualität der Bilddaten B auswerten kann. Im Folgenden wird diese IQA-Einheit 20 genauer beschrieben. In diesem Beispiel werden rekonstruierte MR-Bilder als Bilddaten B für die IQA-Einheit 20 verwendet. Es könnten aber auch direkt die Rohdaten oder bearbeitete Rohdaten als Bilddaten B verwendet werden.

Eine Bedienung der zentralen Steuereinrichtung 13 kann über ein Terminal 11 mit einer Eingabeeinheit 10 und einer Anzeigeeinheit 9 erfolgen, über das somit auch das gesamte Magnetresonanztomographie-System 1 durch eine Bedienperson bedient werden kann. Auf der Anzeigeeinheit 9 können auch Magnetresonanztomographie-Bilder angezeigt werden, und mittels der Eingabeeinheit 10, gegebenenfalls in Kombination mit der Anzeigeeinheit 9, können Messungen geplant und gestartet und insbesondere Steuerprotokolle P ausgewählt und gegebenenfalls modifiziert werden.

Dieses Terminal kann Teil des MRT-Systems 1 sein und durchaus von der Steuereinrichtung 13 gesteuert werden können z. B. zur Ausgabe von Informationen zur Qualität der aufgenommenen MR-Bilder oder von Warnungen falls die Qualität mangelhaft ist.

Das erfindungsgemäße Magnetresonanztomographie-System 1 und insbesondere die Steuereinrichtung 13 können darüber hinaus noch eine Vielzahl von weiteren, hier nicht im Einzelnen dargestellten, aber üblicherweise an derartigen Anlagen vorhandenen Komponenten aufweisen, wie beispielsweise eine Netzwerkschnittstelle, um das gesamte System mit einem Netzwerk zu verbinden und Rohdaten und/oder Bilddaten bzw. Parameter-karten, aber auch weitere Daten, wie beispielsweise patientenrelevante Daten oder Steuerprotokolle, austauschen zu können.

Figur 2 zeigt ein Ausführungsbeispiel für eine erfindungsgemäße IQA-Einheit 20 und eine Konfigurationseinheit 25. Die IQA-Einheit 20 umfasst einen IQA-Algorithmus 21, der die Qualität von eintreffende Bilddaten B bestimmt und ein Ergebnis E (hier in Form eines Bildstapels dargestellt) ausgibt, eine Aggregations-Einheit 22, die Ergebnisse des IQA-Algorithmus 21 aggregiert, eine Scoring-Einheit 23, welche einen numerischen Wert (der "Score" S) als ein mögliches Qualitätsmaß S aus den aggregierten Ergebnissen E des IQA-Algorithmus 21 erzeugt und eine Rating-Einheit 24, welche ein "Rating" R als ein mögliches Qualitätsmaß R aus dem Score erzeugt, z. B. eine Aussage, ob die Qualität "gut" oder "schlecht" ist. Die Aggregations-Einheit 22 ist hier vorhanden, weil der IQA-Algorithmus 21 in diesem Beispiel als Ergebnis E Aussagen über 2D-Schichten eines 3D-Bildes liefert und diese zu einer vereinigten Aussage über das 3D-Bild zusammengefasst werden sollen. Die Scoring-Einheit 23 kann in der Aggregations-Einheit 22 integriert sein, so dass diese direkt einen Score S als Qualitätsmaß ausgibt. Wird nur ein numerischer Wert benötigt, dann kann auf die Rating-Einheit 24 verzichtet werden.

Über die Konfigurations-Einheit 25 können Komponenten der IQA-Einheit 20 konfiguriert werden, z. B. ein geeigneter IQA-Algorithmus ausgewählt werden. In diesem Beispiel werden Aggregations-Daten A und Threshold-Daten T verwendet, um die Aggregation der Ergebnisse E zu konfigurieren und einen Grenzwert für die Rating-Einheit 24 vorzugeben ab wann die Qualität mangelhaft ist. Würde beispielsweise ein Score S erzeugt, der die Bildqualität zwischen 0% (schlechtest) und 100% (optimal) angibt, so kann als Threshold-Daten T z. B. 30% vorgegeben werden. Liegt nun ein Score S unter 30%, so würde die Rating-Einheit 24 die Bilddaten B als "schlecht" interpretieren, ansonsten als "gut". Selbstverständlich kann es weitere Abstufungen geben.

Über eine Konfigurations-Benutzerschnittstelle 26, die mit der Konfigurations-Einheit 25 kommuniziert, kann ein Benutzer vorgeben, welche Komponenten der IQA-Einheit 20 in welcher Weise konfiguriert werden sollen.

Wenn ein Steuerprotokoll P (s. Figur 1) ausgeführt wird, werden während der Erfassung der Bilddaten B diese parallel oder seriell mit dem Bildrekonstruktionsprozess in die IQA-Einheit 20 eingespeist. Die Parameter des Steuerprotokolls P werden idealerweise automatisch überprüft, um festzustellen, ob die Einstellung für die verwendeten IQA-Algorithmen 21 anwendbar ist. Zur Generierung des Qualitätsmaßes S, R wird, falls zutreffend und wie konfiguriert, ein Schwellwert angewendet. Zusätzlich zu den rechnerischen Konfigurationsoptionen kann bevorzugt auch die Ausgabe konfiguriert werden: Es kann angeboten werden, entweder aggregierte Qualitätsmaße S, R oder die Bildserien der diagnostischen Bildqualitätsklasse oder beides anzuzeigen.

Eine bevorzugte IQA-Einheit 20 kann sowohl eine anatomiespezifische als auch eine indikationsspezifische Bildqualitätsbeurteilung besitzen, um die Anforderungen eines Radiologen für das Lesen von Bildern vollständig zu modellieren. In einer bevorzugten Ausführungsform könnten Besonderheiten der Patienten-Compliance bzw. Zustandsspezifische Besonderheiten (z. B. Demenz, Parkinson-Krankheit, Fötus) oder situationsbezogene Zustände (z. B. zeitkritischer Notfall) von der IQAA berücksichtigt werden, um die Erkennungs-/Akzeptanzschwelle fallspezifisch anzupassen.

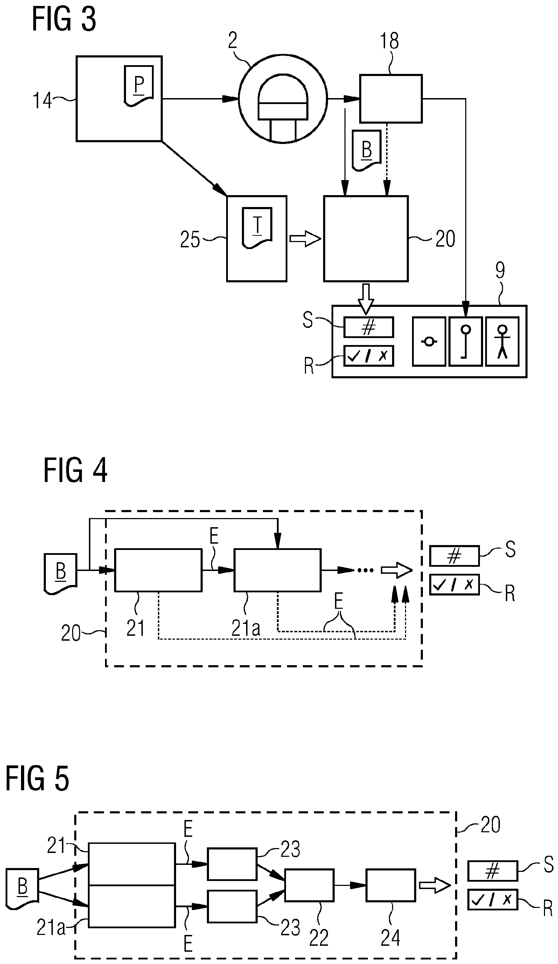

Figur 3 zeigt die Funktionsweise der IQA-Einheit 20 bzw. den Ablauf einer Qualitätsbestimmung. Ein Magnetresonanzscanner 2 erhält Steuerdaten gemäß einem Steuerprotokoll P von einer Sequenzsteuereinheit 14 für eine Bildaufnahme. Die Bilddaten B können direkt als Rohdaten (durchgezogener Pfeil) oder rekonstruierte Bilder einer Rekonstruktionseinheit 18 (gestrichelter Pfeil) an die IQA-Einheit 20 weitergegeben werden. Die IQA-Einheit 20 wird mittels einer Konfigurations-Einheit 25 konfiguriert, welche hier Vorgaben für Threshold-Daten T direkt aus dem Steuerprotokoll P automatisch ableitet. Als Resultat erhält der Benutzer rekonstruierte Bilder der Rekonstruktionseinheit 18 sowie einen Score S und ein Rating R zu diesen Bildern als Qualitätsmaß S, R.

Figur 4 zeigt eine serielle Kombination von IQA-Algorithmen 21, 21a in einer IQA-Einheit 20. Die IQA-Einheit 20 kann zwei oder mehr IQA-Algorithmen 21, 21a enthalten, die in einer Kombination miteinander verwendet werden. In dieser Figur ist eine serielle Kombination dargestellt, in der ein erster IQA-Algorithmus 21 verwendet, der die Qualität von Bilddaten B bestimmt und das Ergebnis E an einen zweiten IQA-Algorithmus 21a weitergibt. Zeigt das Ergebnis an, dass die Qualität der Bilddaten nicht mangelhaft ist, so würde der zweite IQA-Algorithmus 21a die Qualität bestimmen. Dies kann mit weiteren IQA-Algorithmen fortgesetzt werden, was durch die Punkte angedeutet wird. In diesem Beispiel fließen die Ergebnisse E des ersten und zweiten IQA-Algorithmus 20, 21a in das Qualitätsmaß S, R ein welches hier einen Score S und ein Rating R umfasst.

Figur 5 zeigt eine parallele Kombination von zwei IQA-Algorithmen 21, 21a in einer IQA-Einheit 20. Die IQA-Einheit 20 kann durchaus noch mehr IQA-Algorithmen 21, 21a enthalten, die weiter parallel oder seriell (s. Figur 4) in einer Kombination verwendet werden. Die beiden IQA-Algorithmen 21, 21a erhalten und verarbeiten die Bilddaten B hier parallel und jedes der beiden Ergebnisse E wird in eine individuelle Scoring-Einheit 23 weitergegeben. Die Ausgaben dieser Scoring-Einheiten 23 werden in einer Aggregations-Einheit 22 zusammengefasst und ein zusammengefasster Score S gebildet, der an eine Rating-Einheit 24 weitergegeben wird. Der zusammengefasste Score S und das Rating R ergeben dann das Qualitätsmaß S, R. Es ist zu beachten, dass die (ggf. angepasste) Aggregations-Einheit 22 auch zur Zusammenfassung der Ergebnisse der IQA-Algorithmen 21, 21a verwendet werden könnte und die zusammengefassten Ergebnisse E in eine gemeinsame Scoring-Einheit 23 gegeben werden könnten. Es könnten auch Scores S und Ratings R für jeden einzelnen IQA-Algorithmus 21, 21a ohne eine Zusammenfassung ausgegeben werden.

In den Figuren 4 und 5 können die IQA-Algorithmen 21, 21a konfiguriert werden wie in Figur 2 angedeutet, wobei die Konfigurations-Einheit 25 dazu so ausgestaltet sein sollte, dass die verwendeten IQA-Algorithmen 21, 21a ausgewählt werden können, ggf. ihre Reihenfolge angepasst werden kann und die Verarbeitung ihrer Ergebnisse für jedes Ergebnis individuell eingestellt werden kann.

Die beiden IQA-Algorithmen 21, 21a könnten individuelle Qualitätsbewertungen und diagnostische Bildqualitätsklassen berechnen, die auch angezeigt werden können, z. B. in der Anzeigeeinheit 9 der Figur 1. Die IQA-Einheit generiert bevorzugt ein zusammengefasstes Qualitätsmaß S, R, das eine aggregierte Bildqualitätsbewertung oder eine aggregierte diagnostische Bildqualitätsklasse umfasst, basierend auf den Ergebnissen aller konfigurierten IQA-Algorithmen 21, 21a für jede erfasste Bildserie. Die Aggregationsmethode für die Zusammenfassung der Bildqualität ist bevorzugt in einer Konfigurations-Benutzerschnittstelle 26 (s. Figur 2) konfigurierbar. Verfügbare Aggregationsverfahren können z. B. die Berechnung des ausgeglichenen oder gewichteten Durchschnitts, Medianwerts, Minimums oder Maximums von Qualitätsbewertungen der einzelnen konfigurierten IQA-Algorithmen 21, 21a umfassen.

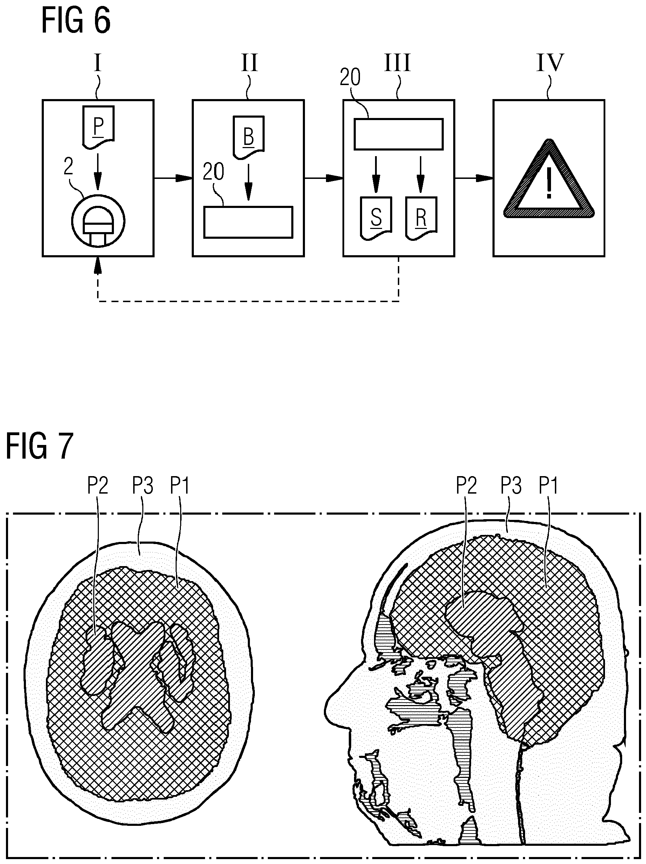

Figur 6 zeigt ein Blockdiagramm eines Ausführungsbeispiels eines erfindungsgemäßen Verfahrens zur Steuerung eines medizintechnischen bildgebenden Systems 1, z. B. eines MRT-Systems 1 wie in Figur 1 dargestellt.

In Schritt I wird ein Steuerprotokoll P für eine Aufnahme von Bilddaten B mit einer Aufnahmeeinheit 2 des medizintechnischen bildgebenden Systems 1 appliziert.

In Schritt II werden die Bilddaten an eine IQA-Einheit 20 gesendet, z. B. eine IQA-Einheit 20 wie sie vorangehend beschrieben wurde.

In Schritt III wird automatisch ein Qualitätsmaß S, R für die Qualität der aufgenommenen Bilddaten B mittels der IQA-Einheit 20 der Steuereinrichtung 13 ermittelt.

In Schritt IV wird das medizintechnische bildgebenden System 1 basierend auf dem Qualitätsmaß S, R gesteuert. Hier ist mit einem Warnschild angedeutet, dass in dem Fall, dass das ermittelte Qualitätsmaß S, R außerhalb eines vorgegebenen Bereichs liegt, eine Warnung von dem medizintechnischen bildgebenden System 1 an einen Benutzer ausgegeben wird. Es könnte aber auch die Aufnahme der Bilddaten B mit geänderten Aufnahmeparametern wiederholt werden oder basierend auf dem Qualitätsmaß S, R eine Anzahl einzelner Schichten oder Segmente neu erfasst und in ursprünglich aufgenommene Bilddaten B eingebettet werden.

Figur 7 zeigt eine räumliche Priorisierung von Bereichen in einem menschlichen Kopf. Die netzartig schraffierten Bereiche sind Bereiche hoher Priorität P1, die schräg schraffierten Bereiche sind Bereiche mittlerer Priorität P2, und die nicht schraffierten Bereiche sind Bereiche niedriger Priorität P3.

Mittels dieser Priorisierung kann ein gewichtetes Qualitätsmaß (S, R) generiert werden, in dem die Qualität der Bereiche hoher Priorität P1 höher bewertet wird als die Qualität der Bereiche mit mittlerer oder niedriger Priorität P2, P3.

Es wird abschließend noch einmal darauf hingewiesen, dass es sich bei den vorhergehend detailliert beschriebenen Verfahren sowie bei dem dargestellten MRT-System 1 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können. Die Angabe "eine Anzahl" bedeutet, "mindestens ein(e)".

## Patentansprüche

1. Steuereinrichtung (13) zur Steuerung eines medizintechnischen bildgebenden Systems (1), wobei die Steuereinrichtung (13) eine IQA-Einheit (20) umfasst, welche eine Anzahl von IQA-Algorithmen (21, 21a) enthält und zum Generieren eines Qualitätsmaßes (S, R) nach oder während der Applikation eines Steuerprotokolls (P) für eine Aufnahme dieser Bilddaten (B) durch das medizintechnische bildgebende System (1) ausgelegt ist, wobei das Qualitätsmaß (S, R) die Qualität der Bilddaten (B) bewertet, und wobei die Steuereinrichtung (13) dazu ausgelegt ist, basierend auf dem Qualitätsmaß (S, R) automatisch das medizintechnische bildgebende System (1) zu steuern.

2. Steuereinrichtung nach Anspruch 1, wobei zumindest ein IQA-Algorithmus (21, 21a) aus der Gruppe umfassend
- IQA-Algorithmen (21, 21a) zur Signal-Rausch-Berechnung oder Kontrast-Rausch Berechnung,
- Detektionsalgorithmen zum Auffinden von Merkmalen, die mit einer bestimmten Art von Bildqualitätsproblemen verbunden sind,
- IQA-Algorithmen (21, 21a) zur Signalverarbeitung von Zeitreihen, die von Sensoren oder (Teil-)Bilddaten-Messungen erstellt wurden, die direkt oder indirekt verschiedene Bewegungsmerkmale eines Patienten (Herzschlag, Atemzyklus, Darmbewegung, Körperbewegung) messen und bestimmen, mit welcher Wahrscheinlichkeit die analysierten Zeitreihen mit Bildqualitätsproblemen korrelieren,
- IQA-Klassifikationsalgorithmen, die eine Anzahl von Bildqualitätsbewertungsklassen bereitstellen.
- IQA-Regressionsalgorithmen, die eine kontinuierliche Bewertung der Bildqualität innerhalb eines endlichen Wertebereichs liefern,
- IQA-Algorithmen (21, 21a) zur Qualitätsbeurteilung von unbearbeiteten 2D oder 3D K-Raum-Bildern oder räumlichen 2D- oder 3D-Bildern,
- IQA-Algorithmen (21, 21a) zur Qualitätsbeurteilung für eine zeitaufgelöste Bildgebung,
ausgewählt ist,
bevorzugt wobei zumindest ein Teil der IQA-Algorithmen (21, 21a) Informationen zu Spezifikationen für die Anwendbarkeit aufweist und/oder mittels der Änderung von Werten vorgegebener Konfigurationsparameter eingestellt werden kann, bevorzugt die Sensitivität und/oder Spezifität der jeweiligen IQA-Algorithmen (21, 21a).

3. Steuereinrichtung nach einem der vorangehenden Ansprüche, umfassend eine Konfigurations-Einheit (25) ausgelegt zum Empfang und/oder zum Konfigurieren von Konfigurations-Werten (A, T) und zur Einstellung der IQA-Einheit (20), insbesondere eines IQA-Algorithmus (21, 21a) oder anderer Komponenten zur Berechnung des Qualitätsmaßes (S, R), mit den Konfigurations-Werten (A, T),
bevorzugt wobei die Konfigurationseinheit (25) und die IQA-Einheit (20) dazu ausgestaltet sind, dass eine Anzahl der eingesetzten IQA-Algorithmen (21, 21a) basierend auf einer Benutzereingabe und/oder basierend auf einem bei der Aufnahme der Bilddaten (B) verwendeten Steuerprotokoll (P) konfiguriert oder zusammengestellt wird und/oder dass die Weiterverarbeitung von Ergebnissen (E) einer Anzahl von IQA-Algorithmen (21, 21a) konfiguriert wird.

4. Steuereinrichtung nach Anspruch 3, umfassend eine Konfigurations-Benutzerschnittstelle (26), über die ein Benutzer Konfigurations-Werte (A, T) für die Konfigurations-Einheit (25) generieren und in diese eingeben kann und/oder zur Generierung des Qualitätsmaßes (S, R) eine Anzahl von IQA-Algorithmen (21, 21a) aus einer Mehrzahl von IQA-Algorithmen (21, 21a) auswählen kann, insbesondere individuell für eine Anzahl von Steuerprotokollen (P),
bevorzugt wobei dem Benutzer über die Konfigurations-Benutzerschnittstelle (26) für den Einsatz mit einem Steuerprotokoll (P) eine spezifische Anzahl von möglichen anwählbaren IQA-Algorithmen (21, 21a) anzeigt wird, die nach oder während der Applikation dieses Steuerprotokolls (P) für die Ermittlung des Qualitätsmaßes (S, R) verwendet werden sollen, bevorzugt wobei dazu eine automatisierte Prüfung der Kompatibilität mit dem Steuerprotokoll (P) durchgeführt wird, die Spezifikationen des Steuerprotokolls (P) mit vorgegebenen Spezifikationen für die Anwendbarkeit der IQA-Algorithmen (21, 21a) abgleicht und bevorzugt nur kompatible IQA-Algorithmen (21, 21a) anzeigt und/oder wenn die Prüfung der Kompatibilität mit dem Steuerprotokoll eine Inkompatibilität eines IQA-Algorithmus (21, 21a) mit einem bestimmten Steuerprotokoll (P) anzeigt einem Benutzer Rückmeldung gibt, welche minimalen Protokollmodifikationen zu einer Kompatibilität mit welchem zusätzlichen IQA-Algorithmus (21, 21a) führen würden.

5. Steuereinrichtung nach einem der vorangehenden Ansprüche, wobei die Steuereinrichtung (13), insbesondere deren IQA-Einheit (20) oder eine Anzahl von IQA-Algorithmen (21, 21a) der IQA-Einheit (20) oder die Konfigurationseinheit (25), dazu ausgelegt ist, mindestens einen Konfigurations-Wert (A, T) basierend auf einem aktuell applizierten Steuerprotokoll (P) zu ändern und bevorzugt eine Detektionsschwelle der IQA-Einheit (20), insbesondere eines IQA-Algorithmus (21, 21a) der IQA-Einheit (20), einzustellen und/oder basierend auf einem aktuell applizierten Steuerprotokoll (P) eine Anzahl von IQA-Algorithmen (21, 21a) auszuwählen, die in der IQA-Einheit (20) zur Ermittlung des Qualitätsmaßes (S, R) verwendet werden sollen.

6. Steuereinrichtung nach einem der vorangehenden Ansprüche, wobei die Steuereinrichtung (13), insbesondere die IQA-Einheit (20) oder die Konfigurationseinheit (25), dazu ausgelegt ist, basierend auf einem aktuell applizierten Steuerprotokoll (P) die Relevanz von Datenpunkten in den Bilddaten (B) für eine mit diesem Steuerprotokoll (P) verbundene medizintechnische Untersuchung in einer Relevanzkarte numerisch zu erfassen, insbesondere Datenpunkte zur Darstellung von Organen oder Körperbereichen, bevorzugt wobei basierend auf dieser Relevanzkarte ein räumlich gewichtetes Qualitätsmaß (S, R) erstellt wird, bevorzugt wobei Datenpunkte, die für eine vorgegebene Untersuchung weniger relevant sind, mit einer geringeren Gewichtung in dem Qualitätsmaß (S, R) berücksichtigt werden als Datenpunkte, die für die vorgegebene Untersuchung relevanter sind.

7. Steuereinrichtung nach einem der vorangehenden Ansprüche, wobei die IQA-Einheit (20) eine Mehrzahl von IQA-Algorithmen (21, 21a) umfasst, und dazu ausgelegt oder dazu einstellbar ist, dass basierend auf zwei oder mehr IQA-Algorithmen (21, 21a) ein Qualitätsmaß (S, R) für die Qualität der Bilddaten (B) einer Bildaufnahme ermitteln, bevorzugt wobei
- in einer parallelen Konfiguration eine Mehrzahl von IQA-Algorithmen (21, 21a) ein jeweils individuelles Ergebnis (E) aus den Bilddaten (B) generieren und basierend auf diesen Ergebnissen (E) ein zusammenfassendes Qualitätsmaß (S, R) ermittelt wird, bevorzugt ein ausgeglichener oder gewichteter Durchschnitt, ein Medianwert, ein Minimum oder ein Maximum der Ergebnisse (E),
- in einer seriellen Konfiguration zunächst ein erster IQA-Algorithmus (21, 21a) ein Ergebnis (E) generiert und in dem Fall, dass sich das Ergebnis (E) in einem vorbestimmten Unsicherheitsbereich befindet, ein weiterer IQA-Algorithmus (21, 21a) ein weiteres Ergebnis (E) generiert und aus dem Ergebnis (E) des letzten IQA-Algorithmus (21, 21a) oder basierend auf mehreren der Ergebnisse (E) ein Qualitätsmaß (S, R) ermittelt wird,
- eine Untergruppe der IQA-Algorithmen (21, 21a) parallel konfiguriert ist und mit einer Anzahl von anderen IQA-Algorithmen (21, 21a) oder Untergruppen von IQA-Algorithmen (21, 21a) seriell konfiguriert ist,
bevorzugt wobei die Einstellung, welche IQA-Algorithmen (21, 21a) in paralleler Konfiguration und welche in serieller Konfiguration ausgeführt werden, bestimmt wird durch:
- eine Voreinstellung durch einen klinischen Bediener,
- eine Priorität eines Bildqualitätsproblems, auf das ein bestimmter IQA-Algorithmus (21, 21a) der IQA-Einheit (20) abzielt,
- eine Häufigkeit des Auftretens eines Bildqualitätsproblems im klinischen Zentrum,
- verfügbare Rechenressourcen,
- Art und Ressourcenverbrauch der IQA-Algorithmen (21, 21a),
- technische Notwendigkeiten.

8. Steuereinrichtung nach einem der vorangehenden Ansprüche, wobei die IQA-Einheit (20) ein Software-Modul auf einer Verarbeitungshardware der Scannerhardware, auf einem Peripherie-Gerät eines medizintechnischen bildgebenden Systems, insbesondere eines MRT-Systems, eines CT-Systems oder eines PET-Systems, oder in einer Cloud ist und bevorzugt eine Konfigurations-Benutzerschnittstelle als Software-Modul in einer grafischen Scanner-Benutzerschnittstelle integriert ist.

9. Verfahren zur Steuerung eines medizintechnischen bildgebenden Systems (1), insbesondere eines MRT-Systems (1), eines CT-Systems oder eines PET-Systems, mit einer Steuereinrichtung (13) nach einem der vorangehenden Ansprüche, umfassend die Schritte:
- Applizieren eines Steuerprotokolls (P) für eine Aufnahme von Bilddaten (B) mit einer Aufnahmeeinheit (2) des medizintechnischen bildgebenden Systems (1),
- automatisches Ermitteln eines Qualitätsmaßes (S, R) für die Qualität der aufgenommenen Bilddaten (B) mittels der IQA-Einheit (20) der Steuereinrichtung (13),
- Steuern des medizintechnischen bildgebenden Systems (1) basierend auf dem Qualitätsmaß (S, R), bevorzugt wobei in dem Fall, dass das ermittelte Qualitätsmaß (S, R) außerhalb eines vorgegebenen Bereichs liegt, eine Warnung von dem medizintechnischen bildgebenden System (1) an einen Benutzer ausgegeben wird und/oder die Aufnahme der Bilddaten (B) mit geänderten Aufnahmeparametern wiederholt wird und bevorzugt basierend auf dem Qualitätsmaß (S, R) eine Anzahl einzelner Schichten oder Segmente neu erfasst und in ursprünglich aufgenommene Bilddaten (B) eingebettet werden.

10. Verfahren nach Anspruch 9, wobei die Bilddaten (B) Daten einer DICOM-Serie sind und/oder MR-Spektroskopiemessungen, quantitative T1- oder T2-Karten oder MR-Fingerprinting-Karten,
bevorzugt wobei das Qualitätsmaß (S, R) für die gesamte DICOM-Serie, ein 3D-Bild, 3D Rohdaten, ein 2D-Bild, 2D-Rohdaten, eine Anzahl von Schichten in einem Stack, ein Segment eines Bildes oder ein K-Raum-Segment generiert wird.

11. Verfahren nach Anspruch 9 oder 10, wobei in dem Fall, dass eine Eingabedimensionsspezifikation der IQA-Einheit (20) nicht mit der Dimension der erfassten Bilddaten (B) übereinstimmt, eine Aggregation von Daten durchgeführt wird, bevorzugt wobei die IQA-Einheit (20) einen Konfigurationsparameter aufweist, der angibt, wie die Aggregation durchgeführt werden soll und dieser Konfigurationsparameter über eine Konfigurations-Benutzerschnittstelle (26) konfiguriert werden kann, wobei die IQA-Einheit (20) bevorzugt eine Auswahl von Score-Aggregationsoptionen der Gruppe Berechnung des Durchschnitts, Berechnung des Medians, Berechnung des Minimums und Berechnung des Maximums von Qualitätsmaßen (S, R) von Teilbildern und/oder Ergebnissen von IQA-Algorithmen (21, 21a) beinhaltet, die im Rahmen der Konfiguration ausgewählt werden können.

12. Medizintechnisches bildgebendes System, insbesondere ein MRT-System, ein CT-System oder ein PET-System umfassend eine Steuereinrichtung nach einem der Ansprüche 1 bis 8 und bevorzugt ausgelegt zur Durchführung eines Verfahrens nach einem der Ansprüche 9 bis 11.

13. Medizintechnisches bildgebendes System, nach Anspruch 12 umfassend eine Anzeigeeinheit, wobei die Steuereinrichtung dazu ausgelegt ist, das Qualitätsmaß (S, R) oder auf dem Qualitätsmaß (S, R) basierende Bewertungen darzustellen und oder andere Ergebnisse der IQA-Einheit (20) darzustellen, insbesondere rekonstruierte Bilder.

14. Computerprogrammprodukt, umfassend Befehle, die bei der Ausführung des Programms durch einen Computer diesen veranlassen, die Schritte des Verfahrens nach Anspruch 9 bis 11 auszuführen.

15. Computerlesbares Speichermedium, umfassend Befehle, die bei der Ausführung durch einen Computer diesen veranlassen, die Schritte des Verfahrens nach Anspruch 9 bis 11 auszuführen.
